# EUROPEAN PATENT SPECIFICATION

(11) **EP 0 359 919 B1**
(45) Date of publication and mention of the grant of the patent: **28.02.1996**
(21) Application number: 89111748.3
(22) Date of filing: 28.06.1989
(51) Int. Cl.: C12N 15/31, A61K 39/02, C12N 1/21, C07K 14/30, C12P 21/02

(54) **Recombinant mycoplasma hyopneumoniae antigen and uses therefor**
Rekombinantes hyopneumoniae Antigen und dessen Verwendung
Antigène recombinant de mycoplasma hyopneumoniae et utilisations de celui-ci

(30) Priority: 29.06.1988 US 213248; 07.04.1989 US 334586; 21.04.1989 US 341968
(43) Date of publication of application: 28.03.1990
(73) Proprietor: ML TECHNOLOGY VENTURES, L.P., New York New York 10080 (US)
(72) Inventor: Faulds, Daryl H., California (US); Brooks, Emily, California (US); Andrews, William H., California (US); Lory, Carol, California (US)
(74) Representative: Perry, Robert Edward

(56) References cited:
- EP-A- 0 196 215
- EP-A- 0 283 840
- WO-A-86/00019
- WO-A-88/00977

## Description

This invention relates to new expression vehicles and to transformed hosts that express recombinant Mycoplasma hyopneumoniae antigens.

The disease caused by Mycoplasma hyopneumoniae (in particular in swine), occurs throughout the world and is a disease associated with the loss of swine. The disease generally results in inefficient, stunted and sickly animals, and affected swine are often prone to secondary infection by opportunistic microorganisms. There have been numerous attempts to provide a vaccine for protecting swine against mycoplasmal pneumonia; however, such vaccines have not been successful.

WO-A-8800977 describes the expression of portions of the genes of Mycoplasma hyopneumoniae encoding antigenic polypeptides A (105 kDa), B (90 kDa), C (85 kDa), D (70 kDa and E (43 kDa).

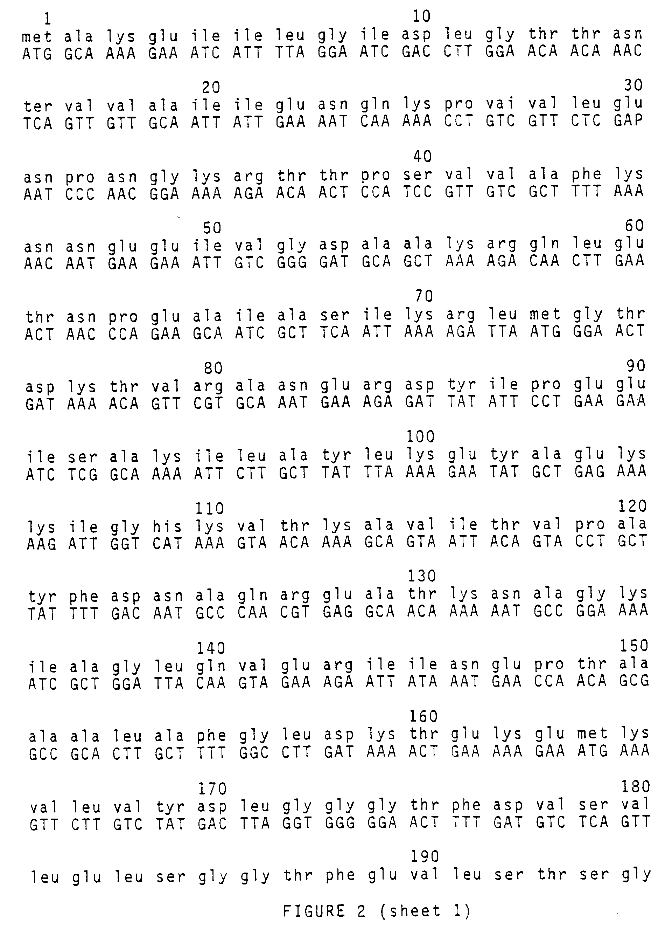

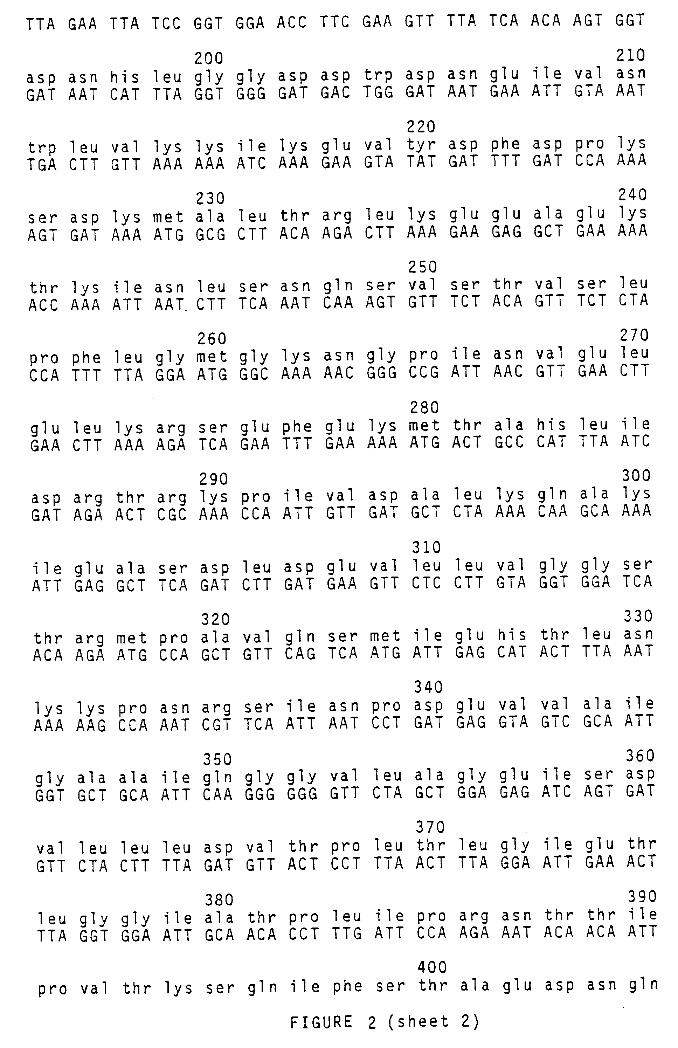

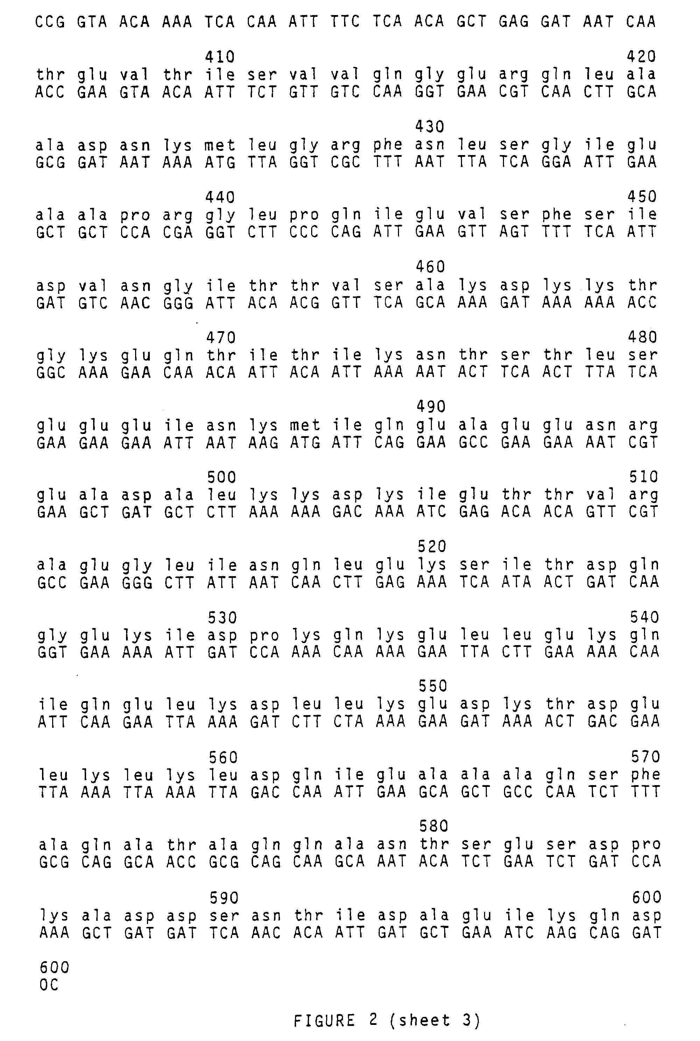

According to a first aspect of the present invention, an expression vehicle includes a DNA sequence as shown in Figure 2, or a portion thereof, which encodes a protein which is capable of eliciting an antibody which recognises an epitope of the Mycoplasma hyopneumoniae 74.5 kDa antigen.

According to a second aspect of the present invention, a host organism is transformed with an expression vehicle as defined above.

The DNA sequence may encode for a protein which is the entire antigen, or a fragment or derivative of the antigen, or a fusion product of the antigen or fragment and another protein, provided that the protein which is produced from such DNA sequence is capable of eliciting an antibody which recognizes an epitope(s) of the M. hyo. antigen. Thus, for example, the DNA sequence may encode for a protein which is a fragment of the 74.5 kDa antigen (a protein having a molecular weight of 43 kDa and which includes a portion of the 74.5 kDa antigen peptide sequence) provided that such fragment is capable of eliciting an antibody which recognizes an epitope(s) of the 74.5 kDa antigen.

Similarly, the DNA sequence may encode for a protein which is a derivative of the antigen e.g., a mutation of one or more amino acids in the peptide chain, as long as such derivative is capable of eliciting an antibody which recognizes an epitope(s) of a M. hyo. antigen as hereinabove described.

The DNA sequence may encode for a protein which protein is a fusion product of (i) a protein which is capable of eliciting an antibody which recognizes an epitope(s) of the noted M. hyo. antigens and (ii) another protein.

As a result, the term "DNA sequence which encodes for a protein which is capable of eliciting an antibody which recognizes an epitope(s) of the noted M. hyo. antigens" encompasses DNA sequences which encode for and/or express in appropriate transformed cells, proteins which may be the appropriate antigen, antigen fragment, antigen derivative or a fusion product of such antigen, antigen fragment or antigen derivative with another protein.

It is also to be understood that the DNA sequence present in the vector when introduced into a cell may express only a portion of the protein which is encoded by such DNA sequence, and such DNA sequence is within the noted terminology, provided that the protein portion expressed is capable of eliciting an antibody which recognizes an epitope(s) of one or more of the noted M. hyo. antigens. For example, the DNA sequence may encode for the entire antigen; however, the expressed protein is a fragment of the antigen. It is also to be understood that the cloning vehicle may include DNA which encodes for more than one M.hyo antigen or fragment.

The appropriate DNA sequence may be included in any of a wide variety of vectors or plasmids. Such vectors include chromosomal, nonchromosonal and synthetic DNA sequences; e.g., derivatives of SV40; bacterial plasmids; phage DNA's; yeast plasmids; vectors derived from combinations of plasmids and phage DNAs, viral DNA such as vaccinia, adenovirus, fowl pox, virus, pseudorabies, etc.

The appropriate DNA sequence may be inserted into the vector by a variety of procedures. In general, the DNA sequence is inserted into an appropriate restriction endonuclease site by procedures known in the art. Such procedures and others are deemed to be within the scope of those skilled in the art.

The DNA sequence in the vector is operatively linked to an appropriate expression control sequence(s) (promoter) to direct mRNA synthesis. As representative examples of such promoters, there may be mentioned: LTR or SV40 promoter, the E. coli lac or trp, the phage lambda PL promoter and other promoters known to control expression of genes in prokaryotic and eukaryotic cells or their viruses. The expression vector also contains a ribosome binding site for translation initiation and a transcription terminator. The vector may also include appropriate sequences for amplifying expression.

In addition, the expression vectors preferably contain a gene to provide a phenotypic trait for selection of transformed host cells such as dihydrofolate reductase or neomycin resistance for eukaryotic cell culture, or such as tetracycline or ampicillin resistance in E. coli.

The vector containing the appropriate DNA sequence as hereinabove described, as well as an appropriate promoter or control sequence, may be employed to transform an appropriate host to permit the host to express the protein. As representative examples of appropriate hosts, there may be mentioned: bacterial cells, such as E. coli, Salmonella typhimurium, fungal cells, such as yeast; animal cells such as CH0 or Bowes melanoma; plant cells, etc. The selection of an appropriate host is deemed to be within the scope of those skilled in the art from the teachings herein.

As hereinabove indicated, the expression vehicle including the appropriate DNA sequence inserted at the selected site may include a DNA or gene sequence which is not part of the gene coding for the protein which is capable of eliciting antibodies which recognize epitopes of the noted M. hyo. antigen(s). For example, the desired DNA sequence may be fused in the same reading frame to a DNA sequence which aids in expression or improves purification or permits expression of the appropriate protein or increases the immunogenicity.

When seeking to develop a vaccine neutralizing or protective antibodies could be targeted towards discontinuous, conformation-dependent epitopes of the native antigen. One must therefore consider whether the protein obtained from the recombinant expression system might have a three dimensional structure (conformation) which differs substantially from that of the original protein molecule in its natural environment. Thus, depending on the immunogenic properties of the isolated proteins, one might need to renature it to restore the appropriate molecular conformation. Numerous methods for renaturation of proteins can be found in the scientific literature and include; 1) denaturation (unfolding) of improperly folded proteins using agents such as alkali, chaotropers, organic solvents, and ionic detergents followed by a renaturation step achieved by dilution, dialysis, or pH adjustment to remove the denaturant, and 2) reconstitution of proteins into a lipid bilayer or liposome to re-create a membrane like environment for the immunogenic protein.

As hereinabove noted, in some cases, even through the DNA sequence included in the cloning vehicle encodes for a specific antigen, the expressed protein may be only a fragment of such antigen. For example, in employing E. coli as a host organism, the codon TGA is a stop codon for E. coli whereby if the DNA sequence in the cloning vehicle includes the codon (TGA) for the amino acid tryptophan, such codon is interpreted in E. coli as a stop codon whereby the entire antigen may not be expressed.

In accordance with another aspect of the present invention, there is provided an expression vehicle which includes (a) at least one DNA sequence for a Mycoplasma protein which includes at least one codon for an amino acid of such protein which is recognized as a stop codon by a host organism to be transformed with the expression vehicle and (b) at least one DNA sequence encoding a t-RNA which binds to the stop codon in the host organism to be transformed and inserts in the nascent Mycoplasma protein an amino acid whereby premature termination of protein synthesis is prevented.

The t-RNA whose DNA sequence is included in an expression vehicle may be a t-RNA which inserts the same amino acid which is encoded by the codon in question in Mycoplasma or one which inserts a different amino acid.

Thus, for example, the additional DNA sequence may encode a t-RNA trp which binds to the stop codon in the transformed host and inserts the amino acid tryptophan into the Mycoplasma protein being synthesized.

Alternatively the additional DNA sequence may encode a t-RNA which binds to the stop codon in the transformed host and replaces the amino acid tryptophan in the expressed protein with a different amino acid, which is preferably tyrosine, phenylalanine, or isoleucine.

Thus, for example, the use of additional DNA in the expression vehicle which encodes a trpT gene, e.g., trpT 176 or trpT 178, which in the host organism encodes a t-RNA trp which binds to the TGA stop codon, inserts tryptophan and thereby prevents premature termination of a Mycoplasma protein which includes the codon TGA (which encodes for the amino acid tryptophan in Mycoplasma) and the expressed recombinant Mycoplasma protein includes the tryptophan encoded by such TGA codon.

If the additional DNA encodes for a variant form of a t-RNA for an amino acid other than tryptophan; for example a tyr t-RNA gene or an Ile t-RNA gene or a pheT gene, which would bind to the TGA codon and insert tyrosine, or isoleucine, or phenylalanine, respectively, then in the expressed Mycoplasma protein the tryptophan normally present in the protein synthesized in Mycoplasma is replaced by an amino acid corresponding to the t-RNA which is encoded by the additional DNA.

In accordance with one embodiment, a cloning vehicle which is to be used for transforming an organism which recognizes the codon TGA as a stop codon (such as E.coli.) may include (in addition to the DNA sequence which encodes for a desired M.hyo. protein having a TGA codon as part of the DNA sequence) an additional DNA sequence which encodes for t-RNA which binds in the host organism to the codon TGA and inserts tryptophan into the M.hyo. protein chain which is produced in the transformed organism. For example, a DNA sequence which encodes for the trpt176 gene may be inserted into the cloning vehicle with the DNA sequence having a TGA codon, which DNA sequence encodes for a desired M. hyo. protein, and the use of such a cloning vehicle in E. coli expresses a desired M. hyo. protein which includes tryptophan. Thus, for example, as shown in Example 3, the 74.5 kDa M. hyo. antigen may be expressed in E. coli by transforming E. coli with a cloning vehicle which includes a DNA sequence which encodes for the 74.5 kDa M.hyo. antigen and a DNA sequence which encodes for the trpT176 gene. As shown in Example 1, transforming E. coli with a cloning vehicle which includes a DNA sequence which encodes for the 74.5 kDa M. hyo. antigen and does not include a DNA sequence which encodes for the trpT176 gene, expresses a fragment of the 74.5 kDa M.hyo. antigen.

In accordance with another aspect of the present invention, there is provided an expression vehicle which includes at least one DNA sequence for a Mycoplasma protein, said at least one DNA sequence having been formed from at least one DNA sequence for a Mycoplasma protein which includes at least one codon for an amino acid of such protein which is recognized as a stop codon by a host organism to be transformed with the expression vehicle, wherein said at least one stop codon has been mutated to another codon which encodes for said amino acid, wherein said another codon is not recognized as a stop codon by the host organism. Through mutation of the at least one stop codon to another codon which is not recognized as a stop codon by the host organism, premature termination of protein synthesis is prevented.

For example, the amino acid at position 211 of the 74.5 kDa antigen is encoded by TGA, which normally encodes for tryptophan, but also is recognized as a stop codon by E. coli. An oligonucleotide containing the TGA codon may be altered through mutagenesis (e.g., site directed mutagenesis) such that the TGA codon is changed to TGG, which also encodes for tryptophan. The oligonucleotide sequence is then substituted into that portion of the DNA sequence of the 74.5 kDa antigen's gene which includes the corresponding TGA codon. In this manner, an expression vehicle may be produced which expresses the full-length 74.5 kDa antigen without an additional DNA sequence encoding for a t-RNA which binds to the TGA codon in the host organism.

In accordance with another aspect of the present invention, there is provided an expression vehicle which includes at least one DNA sequence which encodes for at least one Mycoplasma protein, said at least one DNA sequence having been formed from at least one DNA sequence having at least one codon encoding a first amino acid of such protein, wherein said at least one codon has been mutated to at least one codon or codons encoding an amino acid or amino acids which are different from the first amino acid. The DNA sequence of such an expression vehicle may also include a codon or codons which had been formed by mutating a codon or codon for an amino acid, but which is recognized as a stop codon by the host organism to be transformed to a codon for the amino acid which is not recognized as a stop codon. Alternatively, such an expression vehicle may include, in addition to the at least one DNA sequence hereinabove described, at least one DNA sequence encoding a t-RNA which binds to a stop codon in the host organism to be transformed, if such a stop codon is present, and inserts in the nascent Mycoplasma protein an amino acid whereby termination of protein synthesis is prevented.

An example of such a recombinant variant, is the r116 variant of the 74.5 kDa antigen, which may be expressed by E. coli, as hereinafter described.

Extensive sequence homology has been found between the 74.5 kDa antigen and the E. coli dnaK protein. Two differences in the amino termini which are noteworthy are that valine residues at positions 17 and 27 of the 74.5 kDa M.hyopneumoniae antigen significantly reduce the predicted T-cell recognition character of these regions of the antigen relative to the E. coli dnaK protein. Such protein domains are associated with the efficient presentation of the antigen to the immune system. By the use of site-directed mutagenesis, the valines at positions 17 and 27 may be replaced with cysteine and arginine, respectively. These two changes restore two regions of predicted amphipathic helicity present in the E.coli dnaK protein. A gene or genes for a derivative containing the two amino acid residue substitutions may be transferred into an E.coli expression vector. An example of such an expressed recombinant protein is known as the r116 variant of the 74.5 kDa M.hyopneumoniae antigen, as hereinafter described.

The present invention will be further described with respect to the following examples; however, the scope of the invention is not to be limited thereby. In the Examples, unless otherwise noted, purifications, digestions and ligations are accomplished as described in "Molecular Cloning, a laboratory manual" by Maniatis et al. Cold Spring Harbor Laboratory (1982). In the following examples, unless otherwise indicted, transformations are accomplished by the procedure of Cohen et al, PNAS 69 2110 (1973).

Unless otherwise indicated the M.hyo antigens used in the following examples are obtained from M.hyo as described in EP-A-0283,840, published September 28, 1988.

### Example 1 - 74.5 kda M. hyo antigen

### Preparation of M. hyopneumoniae DNA

Strain P-57223 (obtained from Dr. Charles Armstrong, Purdue University) was grown in 1 liter of Friis medium to a density of approximately 10⁹ to 10¹⁰ color changing units per ml. The cells were harvested by centrifugation and resuspended in 2 ml phosphate buffered saline which brough the total volume to 3.25 ml. The suspension was then mixed with a solution consisting of 24.53 g cesium chloride dissolved in 19.75 ml 10 mM Tris pH 8.0 1 mM EDTA and 1.53 of 10 mg/ml ethidium bromide was added. This was mixed with a solution consisting of 3.87 g cesium chloride dissolved in 2.15 ml 10 mM Tris pH 8.0, 1 mM EDTA, 8.9% Sarkosyl. The resulting suspension was incubated at 65°C for 10 minutes to completely lyse the cells. The DNA was separated by equilibrium buoyant density centrifugation in a Sorvall TV850 rotor at 43,000 rpm for 18 hours, and withdrawn with an 18 gauge needle. This DNA was subjected to two additional buoyant density centrifugations in a Sorvall TV865 rotor at 55,000 rpm for 7 and 18 hours respectively, each time the band of genomic DNA being removed with an 18 gauge needle. The resulting DNA solution was extracted with cesium chloride saturated isopropanol, to remove ethidium bromide, and extensively dialyzed against 10 mM Tris pH 8.0, 1mM EDTA, to remove the isopropanol and cesium chloride.

### Preparation of Genomic Library

A preparative digest of 200 µg genomic DNA of Mycoplasma hyopneumoniae P-57223 was done using 200 units of EcoR1 in a total volume of 1 ml and 250 µl aliquots were removed at 6 min, 25 min, 42 min and 63 min.

The four preparative samples of partially digested Mycoplasma DNA then combined (200 µg) and loaded on to an exponential sucrose gradient. The gradient was centrifuged in a Sorval AH627 rotor at 26 k rpm for 21 hrs at 15°C.

The gradient was then slowly fractioned from the bottom by collecting 15 drop fractions (90 fractions total). 20 µl of each fraction was then run on a 1% agarose gel as described above. Fractions containing DNA fragments smaller than 18 kbp and larger than 15 kbp were pooled (fractions 32-40) and dialized against TE (10 mM Tris.HCl pH 7.5, 1 mM EDTA pH8.0) to remove the sucrose. The DNA (3.5ml) was then precipitated with ethanol and resuspended to about 15 µl (1 mg/ml) and stored at -20°C.

EcoR1 Arms of bacteriophage lambda-dash were obtained from Vector Cloning Systems (StrataGene) and were ligated at a concentration of 200 µg/ml to Mycoplasma target DNA at a concentration of 25 µg/ml in a total volume of 10 µl using T₄ ligase (Boehringer GmbH) at a concentration of 100 units/ml. The ligation reaction was incubated at room temperature for 2 hours. 4 µl of the ligation was then packaged into lambda particles using the in vitro packaging kit Gigapack (StrataGene). The phage was then titered on E. coli strain P2392 (StrataGene) and found to be 7.75 x 10⁵ pfu/ml (3,1 x 10⁵ pfu/µg of lambda-dash).

### Antiserum:

1. Rabbit antiserum. A new Zealand White rabbit was immunized with approximately 10¹¹ color changing units of M. hyopneumoniae strain J (ATCC 25934) in complete Freund's adjuvant (Sigma, St Louis). A booster injection of the same antigen in incomplete Freund's adjuvant (Sigma) was administered two weeks after the First injection. Hyperimmune serum was shown to react with greater than thirty mycoplasma proteins by 1-D gel Western blot analysis and to react with two proteins of approximately 74.5 kDa by 2-D Western blot analysis.
2. Mouse antisera. Monospecific serum was prepared as described above with the exception that DBA/2 mice were immunized with approximately 10 µg of electrophoretically pure 74.5 kDa antigen from strain P-57223 (Dr. C. Armstrong, Purdue University) followed by an equal dose booster immunization. The 74.5 kDa antigen is obtained as described in EP-A-0283,840 published on September 28, 1988. Hyperimmune sera was shown to react with a single mycoplasma 74.5 kDa protein band by 1-D Western Blot analysis and to react with two 74.5 proteins by 2-D gel Western Blot analysis. The 74.5 kDa antigen was prepared as described in EP-A-0 283,840.
3. Pig antisera. Purdue mini-pigs were immunized with 100 µg of electrophoretically pure 74.5 kDa antigen in incomplete Freund's adjuvant (Sigma). An identical booster injection was administered two weeks after the first injection. Hyperimmune sera was shown to react with a single mycoplasma 74.5 kDa protein by both 1-D and 2-D gel Western blot analysis. This protein is identical to one of the two recognized by the hyperimmune mouse serum. These pigs were afforded a measure of protection from mycoplasmal pneumonia by the vaccination.

### Screening of Library

The library was screened with rabbit anti-M. hyopneumoniae (which immunologically recognizes the 74.5 kDa antigen, in addition to other mycoplasma surface proteins). Aproximately 200 recombinants were selected by the initial screen because they reacted immunologically with the rabbit anti-serum. These positives were subsequently screened with mouse anti-74.5 kDa, which was raised against electrophoretically purified antigen. Ten recombinants were selected because they produced material in E. coli which cross-reacted immunologically with the mono-specific serum. Cloning the Gene for the 74.5 kDa Antigen

Based on a partial amino acid sequence of the 74.5 kDa antigen the oligonucleotide families shown below (COD 558 and COD 559) were synthesized. DNA from the 10 immuno-positive recombinants was 
1

Ala--Lys--Glu--Ile--Ile--Leu--Gly--Ile--Asp--Leu--
AAA GAA ATA ATA TTG GG
G G C C C A
T T T
C
COD 558
 
15

Ser--Val--Val--Leu--Ile--Asp--Glu--Asn--Gln--Lys--Pro
GTG  CTG  ATA  GAC  GAA  AA
  A  T      C    T    G
  T         T
  C
COD 559
 prepared, digested with EcoR1, analyzed by gel electrophoresis, and each shown to contain portions of the M. hyopneumoniae genome composed of several EcoR1 restriction fragments. The hybridization conditions are as follows:
Hybridization:
6 X NET
5 X Denhardts
2 X 10⁶ cpm kinased probe
37°C
18 hours
Wash:
6 X NET
0.1% SDS
3 X at room temperature
2 X at 37°C
6 X NET:
1 M NaCl
90 mM Tris pH 7.6
6 mM EDTA

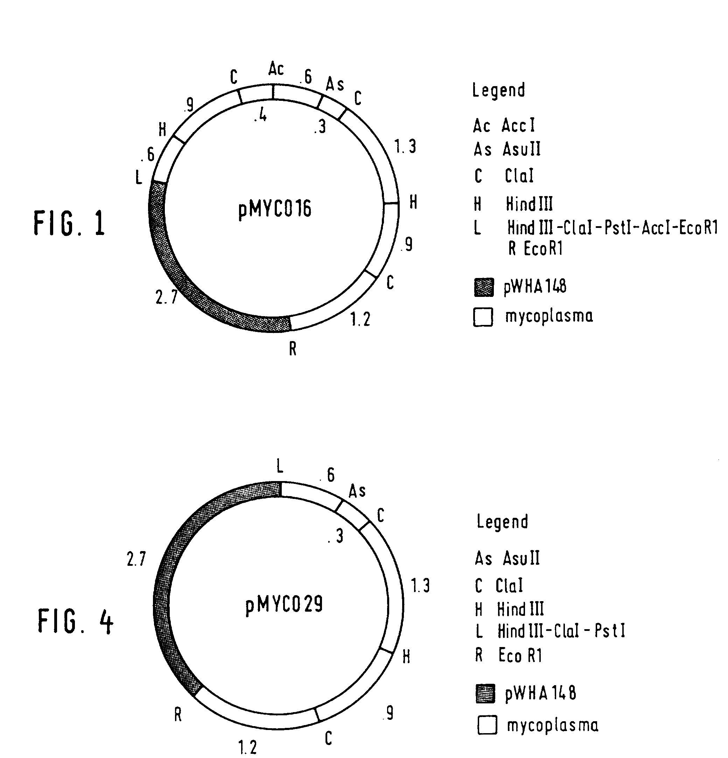

Both COD 558 and COD 559 were shown by Southern blot analysis to hybridize to a 7,800 base pair (7.8 kb) EcoRl restriction fragment present in 6 of the 10 recombinants. In order to subclone this fragment, one recombinant containing the 7.8 kB fragment (in addition to other adjacent fragments) was named lambda 5-5-59, its DNA was prepared and digested with EcoRl, ligated to EcoRl digested pWHA148 and transformed into E.coli strain JM83. One transformant was named pMYCO16; its DNA was prepared and digested with a number of different restriction endonucleases in order to derive the restriction map shown in Fig. 1.

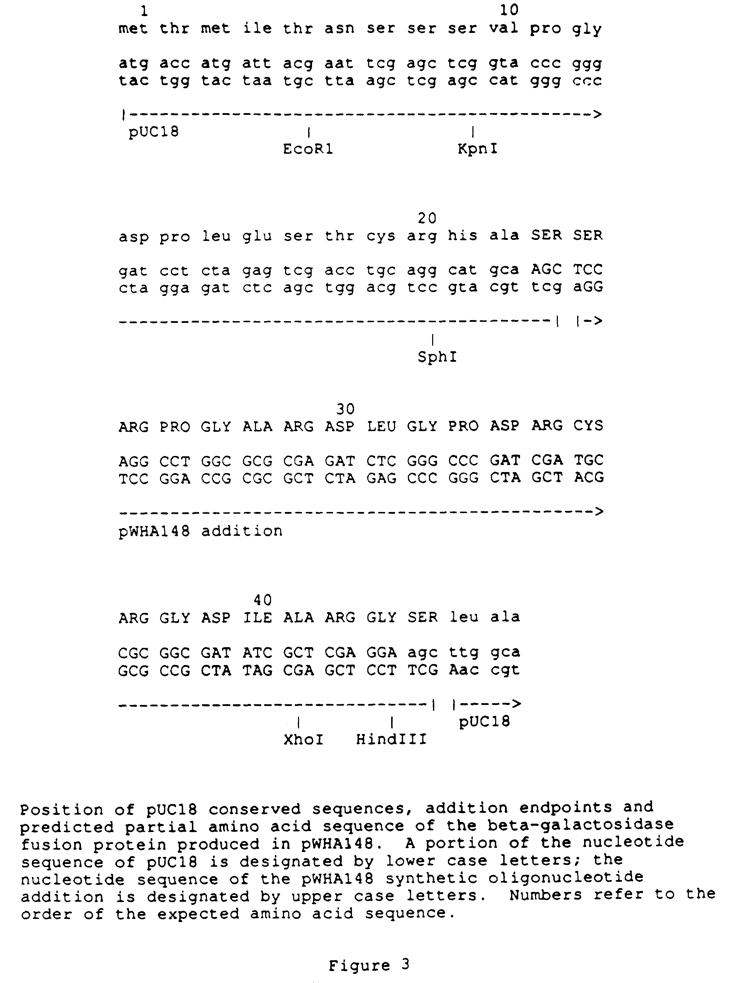

pWHA 148 is prepared by inserting synthetic oligonucleotides into the Hind III site of pUC18. The amino terminal coding sequence of the X-complementing peptide of B-galactosidase is shown in Figure 3, and contains 8 additional restriction sites over the parent pUC18. The oligonucleotide insert into pUC18 is shown in Figure 3 between the Sph1 and Hind III sites.

Both COD 558 and 559 were shown by Southern analysis to hybridize to the 0.6kb AccI-AsuII restriction fragment of pMYCO16. DNA sequence analysis of the 0.6 kb fragment showed: 1) it included a region of homology to COD 558 and COD 559; and 2) all but 3 of the amino acids predicted by the fragment's DNA sequence matched the protein sequence determined for the 74.5 kDa as shown below. 
Protein
sequence: 1---Ala Lys Glu Ile Ile Leu Gly Ile Asp
Clone
sequence: met ala lys glu ile ile leu gly ile asp
Protein
sequence: 11Leu Gly Thr Val Asn Ser Val Val Leu Ile
Clone
sequence:   leu gly thr thr asn ser val val ala ile
Protein
sequence: 21Asp Glu Asn Gln Lys Pro Val Val Leu
Clone
sequence:   ile glu asn gln lys pro val val leu
 Subsequent re-analysis of the actual amino acid sequence indicated that the three mismatches could have been due to ambiguous amino acid identification.

On the restriction map of pMYCO16 (Figure 1) the gene begins within the 0.6 kb AccI-AsuII restriction fragment, extends clockwise within the 0.4 kb AsuII-ClaI, 1.2 kb ClaI, - ClaI, and 1.4 kb ClaI- HindIII fragments, and ends short of the HindIII site.

The DNA-amino acid sequence of the 74.5 kDa gene is shown in Figure 2.

### Expression of M. hyopneumoniae 74.5 kDa antigen gene in E. coli

DNA from pMYCO16 was transformed into E. coli strain CY15000. One transformant was selected, grown in L-broth at 37°C to an OD ₅₅₀ = 2, and the cells harvested by centrifugation. The cells were washed free of contaminating medium components by resuspension in M9 buffer and harvested again by centrifugation. The resulting cell pellet was resuspended at one-fifteenth the original culture volume in a solution consisting of 0.5 mg/ml hen egg-white lysozyme dissolved in 25 mM Tris pH 8.0, 10 mM EDTA; incubated at 25°C for 10 minutes; and sonicated at 4°C for 15 seconds. A portion of the resulting lysate was subjected to polyacrylamide gel electrophoresis and a new 43 kDa protein was identified.

The pMYCO16 43 kDa expression product was shown to react by Western blot with the mouse antiserum raised against the 74.5 kDa M. hyopneumoniae antigen.

The pMYCO16 43 kDa expression product was shown to react by Western blot with the pig antisera raised against the 74.5 kDa M. hyopneumoniae antigen.

### Partial purification of the E. coli Produced 74.5 Antigen Fragment

One E. coli strain CY15000 transformant was selected, grown in one liter of L-broth at 37°C to an OD₅₅₀ = 2, and the cells harvested by centrifugation. The cells were washed free of contaminating medium components by resuspension in M9 buffer and harvested again by centrifugation. The resulting cell pellet was resuspended in 20 ml of a solution consisting of 0.5 mg/ml hen egg-white lysozyme dissolved in 25 mM Tris pH 8.0, 10 mM EDTA; incubated at 25°C for 10 minutes; separated into two aliquots; and each sonicated at 0°C for 60 seconds. The resulting lysate was cleared of insoluble debris by centrifugation at 13,000 x g for 10 minutes at 4°C. Sufficient ammonium sulfate (4.52 g) was added to bring the supernatant to 40% saturation and the insoluble protein was removed by centrifugation. Sufficient ammonium sulfate (1.2 g) was subsequently added to bring the 40% supernatant up to 50% saturation and the insoluble protein was harvested by centrifugation, subjected to polyacrylamide gel electrophoresis, and shown by Western blot analysis (using miniature pig serum prepared as described above) to contain the 43 kDa pMYCO16 expression product. The enriched antigen fraction was dialysed against PBS prior to its use as a vaccine.

### Example 2

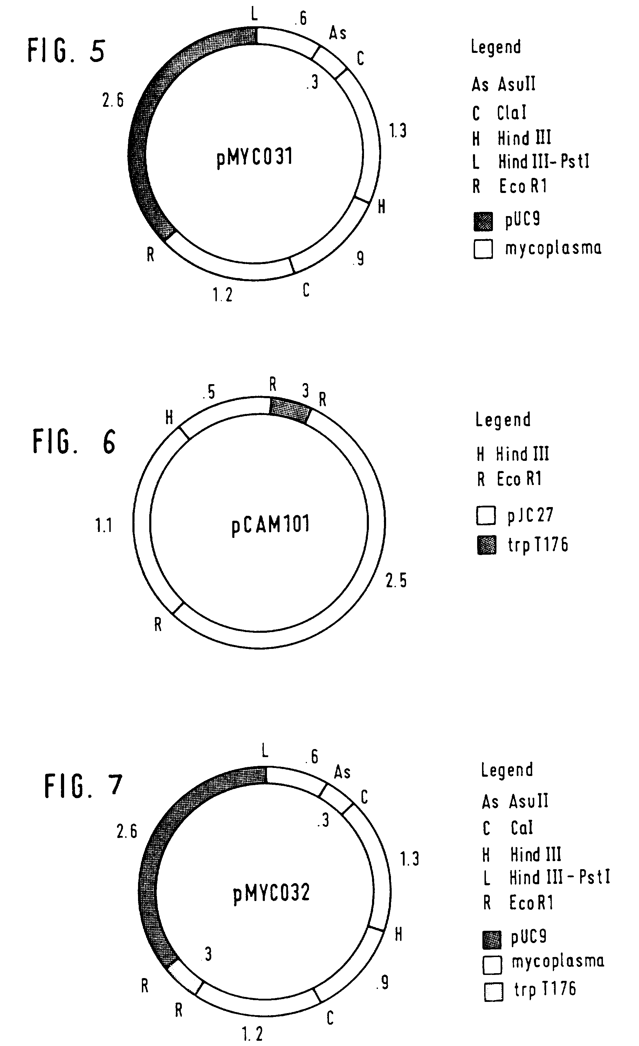

Expression of full length M.hyo. 74.5 kDa antigen in E. Coli:
pMYC016 DNA (Fig. 1) was digested with AccI, treated with Mung Bean nuclease to remove the single stranded AccI tails, re-ligated to delete the 1.9 kb AccI fragment in front of the 74.5 kDa antigen gene and transformed into E.coli strain JM83. One transformant was named pMYC029; its DNA was digested with a number of different restriction endonucleases in order to derive the restriction map shown in Figure 4.
pMYCO29 was subjected to DNA sequence analysis which showed that a spontaneous deletion had occured at the ligation juncture, where two bases were deleted and the PstI site was retained, as shown below (only a portion of the 5' to 3' strands are represented).

pMYCO29 expected: TTGCATGCCTGCAGGTACTTTCTTTTGTCT
PstI
pMYCO29 observed: TTGCATGCCTGCAGGCTTTCTTTTGTCT
PstI

### Construction of pMYCO3l and expression of 74.5 kDa antigen fragment

Because the mycoplasma insert of pMYCO29 is oriented away from the Lac promoter of pWHA148, it was desired to insert the gene into another expression vector, pUC9. The two base deletion enabled the gene for the 74.5 kDa antigen to be placed in the same reading frame as the beta-galactosidase gene of E. coli vector pUC9.

In order to perform this construction, pMYCO29 DNA was digested with PstI and EcoRl, the PstI - EcoR1 fragment containing the entire 74.5 kDa coding sequence was purified, ligated to the PstI and EcoRl digested vector pUC9, and transformed into E. coli strain JM83. One transformant was named pMYCO31 (Figure 5); its DNA was prepared and transformed into E. coli strain CY15000 by the transformation procedure of Example 1.

### Construction of pMYCO32

Plasmid pCAM101 was purchased from James Curran located at University of Colorado, Boulder, Colorado as a convenient source of the trpT176 gene is shown in Figure 6. DNA from pCAM101 was digested with EcoRl, the 0.3 kb EcoRl fragment which contains the trpT176 gene was purified, ligated to EcoRl digested pMYCO31, and transformed into E. coli strain CY15000. One transformant was named pMYCO32 and its restriction map is shown in Figure 7.

### Expression of M. hyopneumoniae 74.5 kDa antigen in E. coli

A CY15000 (pMYCO32) transformant was selected, grown in L-broth, a lysate prepared as previously described, and a portion subjected to polyacrylamide gel electrophoresis. New 75 kDa and 43 kDa proteins were identified by gel electrophoresis which represented approximately 5% and 0.1% of total E. coli protein, respectively. The pMYCO32 75 kDa protein was shown by Western blot to react with the previously described pig antisera raised agains the 74.5 kDa M. hyopneumoniae antigen.

### Example 3

### Use of the recombinant form of Mycoplasma hyopneumoniae 74.5 kDa antigen as a vaccine

A CY15000 (pMYCO32) transformant from Example 2 was selected, grown in M-9 minimal medium in a 14 liter Chemap fermenter to a cell density of 110 O.D. 600, and 120 g (wet weight) of cells were harvested from 500 ml by centrifugation. A suspension was prepared consisting of 2.3 g of cells per 10 ml of PBS containing 12 mM EDTA, 0.5 mg/ml lysozyme. The suspension was incubated at 25°C for 15 minutes, sonicated on ice for 2 minutes in 30 second bursts, centrifuged at 13,000 g for 10 minutes at 4°C, and the soluble fraction reserved as product. A portion of the product was subjected to polyacrylamide gel electrophoresis. The recombinant form of 74.5 kDa antigen made up approximately 25% of the soluble protein and the dosages, based on the amount of 74.5 kDa antigen in the preparation were prepared in PBS at 200 and 1000µg per dose and emulsified on ice with equal volumes of Freund's incomplete adjuvant (Sigma) immediately prior to administration to animals.

### EXAMPLE 4 - Expression of r116 Variant of M.hyopneumoniae 74.5 kDa AntiRen in E. coli

### Construction of M13MYCO102

Plasmid pMYCO31 was used as the source of the 74.5 kDa antigen's gene (see Example 2, Figure 5). M13mp 18 RF DNA (Bethesda Research Labs), a 7253 base pair phage vector (Figure 8), was digested with HindIII (Boehringer Mannheim) and PstI (Bethesda Research Labs), ligated with T4 ligase (Boehringer Mannheim) to HindIII, PstI digested pMYCO31 and transformed into E.coli strain JM103. The DNA from one transformant, M13MYCO102, was digested with restriction endonucleases in order to derive the restriction map shown in Figure 9.

### Construction of M13MYCO107

Based on the DNA sequence of the 74.5 kDa antigen gene, a 96 base long synthetic oligonucleotide, COD 639 (see below) was designed to alter 
11LEU-GLY-THR-THR-ASN-SER-VAL-VAL-ALA-ILE-ILE-GLU

CTT-GGA-ACA-ACA-AAC-TCA-GTT-GTT-GCA-ATT-ATT-GAA
-TGT-TGT-TTG-AGT-ACA-CAA-CGT-TAA-TAA-CTT

23ASN-GLN-LYS-PRO-VAL-VAL-LEU-GLU-ASN-PRO-ASN-GLY

AAT-CAA-AAA-CCT-GTC-GTT-CTC-GAA-AAT-CCC-AAC-GGA
TTA-GTT-TTT-GGA-GCG-CAA-GAG-CTT-TT
COD 639
 two amino acids in the antigen's N-terminus through site directed mutagensis based on the methods of Taylor et al. (1985) Nucl. Acid Res. 13: 8749-8764 and Taylor et al. (1985) Nucl. Acid Res. 13:8764-8785. The mutagenesis with COD 639 changes Val¹⁷ to Cys¹⁷, destroying a DdeI site, and changes Val⁷ to Arg⁷, creating a ThaI site. These changes restore two regions of predicted amphipathic helicity present in the E.coli dnaK protein as hereinabove described.

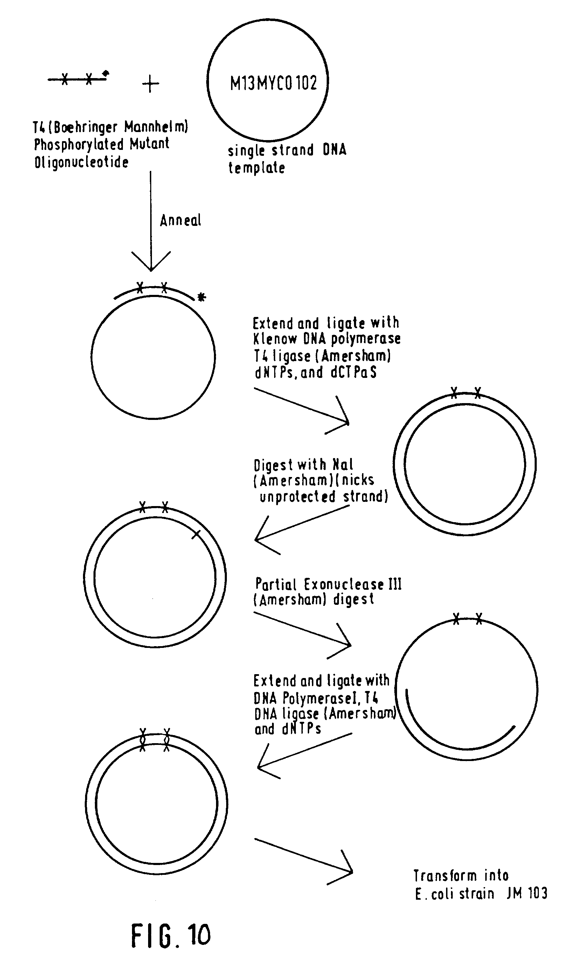

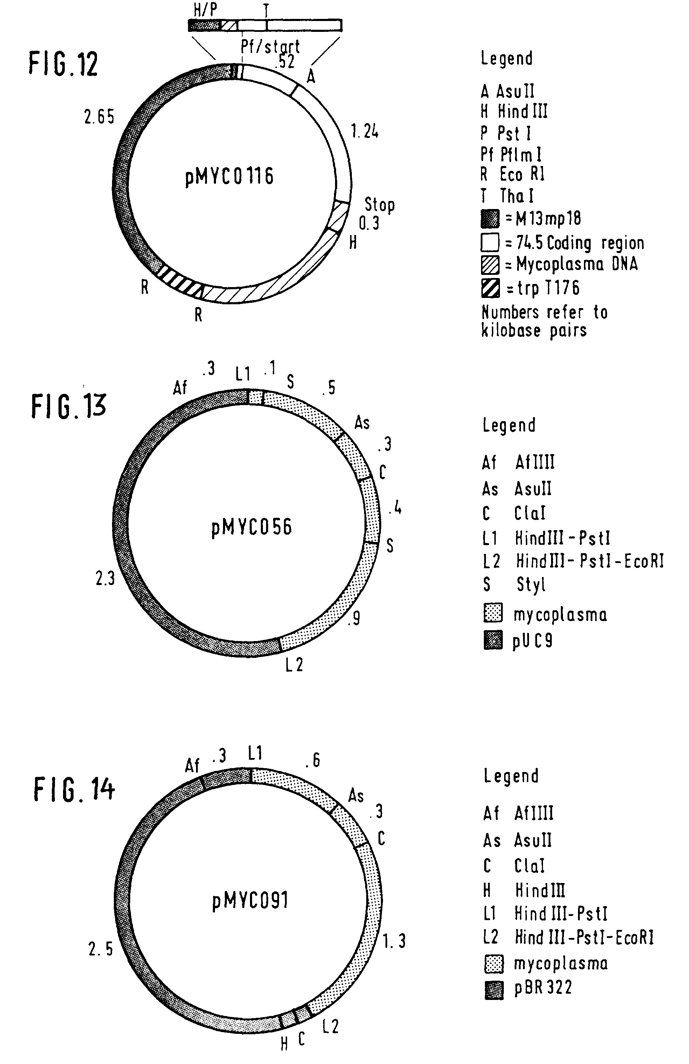

Single strand DNA of M13MYCO102, prepared according to Nakamaye, K. and F. Eckstein (1986) Nucl. Acid. Res. 14:9679-9698, was used as the template in the steps shown in Figure 10 used to generate the recombinant candidate named M13MYCO107; its DNA was prepared and digested with ThaI in order to verify one of the expected mu ational changes. As predicted, M13MYCO107 (Figure 11) has an additional ThaI site which converts a 2 kb fragment into two fragments of 0.4 and 1.6 kb. DNA sequence analysis of M13MYCO107 compared to M13MYCO102 confirmed both changes and indicated that no other changes took place within 70 base paris on either side of COD 639.

### Construction of pMYCO116

Purified pMYCO32 DNA (see Example 2, Figure 7) was digested with PflmI. (New England Biolabs), phosphatased with Calf Intestine Phosphatase (CIP: Boehringer Mannheim), digested with PstI (Promega Biotech) and AsuII (New York Biolabs), and phosphatased with CIP again, ligated to PstI, AsuII digested M13MYCO107 with T4 ligase (Boehringer Mannheim), and transformed into E.coli strain JM83. One transformant was named pMYCO116, its DNA purified and digested with HindIII in order to verify a 2.2 kb insert site. Purified pMYCO116 DNA (see Figure 12) was used to transform E.coli strain CY15000. A purified colony was grown in L-Broth containing 100 pg per ml ampicillin. The cells were harvested by brief centrifugation, resuspended in 8% sucrose, 0.5% Triton X-100, 50 mM EDTA, 10mM Tris pH8.3, and 0.66 mg/ml hen egg white lysozyme (HEL: Sigma) then heated to 100°C briefly. Plasmid DNA from the supernatant was digested with HindIII to verify the presence of the proper insert. ThaI and DdeI digests verified the presence of the new ThaI site and the lack of a DdeI site, converting two fragments of 0.26 and 0.76kb into a single 1.0kb fragment.

### Expression of pMYCO116 Product

Cells of CY15000 (pMYCO116) were harvested, washed, sonicated for 15 seconds in 25 mM Tris pH 8.0, 10 mM EDTA, and 0.5 mg/ml hen egg white lysozyme and centrifuted 10 minutes. Two microliters of the supernatant (soluible fraction) was electrophoresed on a 12% polyacrylamide gel. pMYCO116 produced a 74.5 kDa protein, which comprised approximately 10% of the soluble protein, equivalent in size to that produced by pMYCO32.

Soluble fraction from a 125 ml culture was prepared as described and the 74.5 kDa product was electrophoretically purified. Amino acid sequence analysis of 31 amino terminal residues confirmed the engineered protein and was consistent with Val¹⁷ being changed to Cys¹⁷. However, cysteine is not directly verifiable by protein sequence analysis due to its weak signal. The change Val⁷ to Arg⁷ was confirmed by detection of arginine instead of valine at residue 27.

### EXAMPLE 5 - Preparation and Administration of Vaccine Purification of pMYCO32 Product for Use in a Vaccine

E. coli CY15000 (pMYCO32) was grown in potassium phosphate buffered minimal medium in a 14 liter Chemap fermenter to a cell density of 84 O.D.600.360g (wet weight) of cells were harvested from 4.2 liters. 100g of cells were suspended in 300 ml PBS containing 12 mM EDTA. Cell disruption was accomplished with a Menton-Gaulin homogenizer operating at 5-8,000 psi feed pressure. Up to ten moles EDTA is added per liter harvest material to aid homogenization. The homogenized material is then clarifeid by microfiltration. 50 ml of filtrate was applied to a 100 ml radial flow DEAE column where both the sample and the column were equilibrated with 50 mM NaPO₄, pH 7.0, 2 mM EDTA and the unbound fraction retained as product. This material is sterile filtered. Immediately prior to use, 100 µg./ml PBS may be emulsified for use as a vaccine. A unit dose is equivalent to 100 µg of the purified product.

### Purification of pMYCO116 Product for Use in a Vaccine

E.coli CY15000 (pMYCO116) was grown in potassium phosphate buffered minimal medium in a 14 liter Chemap fermenter to a cell density of 84 O.D.₆₀₀° 360g (wet weight) of cells were harvested from 4.2 liters. 100g of cells were suspended in 300ml PBS containing 12mM EDTA and 0.5mg/ml hen egg white lysozyme, incubated at 25°C for 15 minutes, sonicated on ice for 2 minutes in 30 second bursts, and centrifuged at 13,000g for 10 minutes at 4°C. The soluble fraction was retained. Polyacrylamide gel electrophoresis indicated the 74.5 kDa antigen comprised approximately 10% of the soluble fraction or approximately 2.5g product per liter of fermentation culture. 50ml of soluble fraction was applied to a 100ml radial flow DEAE column where both the sample and the column were equilibrated with 50mM NaPO₄, pH7.0, 2mM EDTA and the unbound fraction retained as a product. Immediately prior to use, 100µg product/ml PBS may be emulsified for use as a vaccine. A unit dose is equivalent to 100µg of the purified product.

### Example 6 - Mutation of TGA Codon Corresponding to Amino Acid 211 of 74.5 kDa Antigen

The amino acid tryptophan at position 211 of the 74.5 kDa antigen gene (see Figure 2, Translation of 74.5 kDa gene) is encoded by TGA. Studies of proteins produced by pMYC031 and pMYC032 showed that the TGA codon terminates peptide chain elongation in E.coli in the absence of the trpT176 suppressor. It has been postulated that changing this codon to TGG by in vitro mutagenesis would allow peptide chain elongation at position 211 in the absence of the trpT176 suppressor.

DNA from pMYC032 was digested with HindIII, the 2.2kb fragment containing the 74.5 kDa antigen's gene was ligated to HindIII digested vector pUC9 using T4 ligase, and transformed into E.coli strain JM83. One transformant was named pMYC056; its restriction map is shown in Figure 13. As expected, pMYC056 produces a prematurely terminated fragment of the 74 kDa antigen.

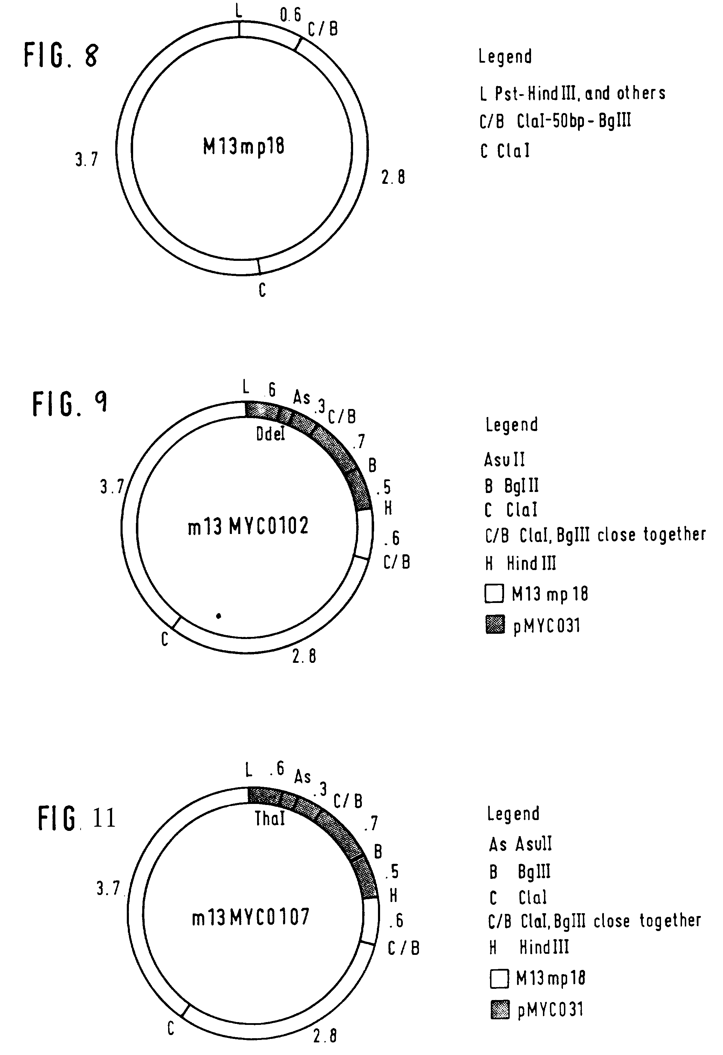

M13mp18 (Figure 8) RF DNA, a 7253 base pair phage vector, was digested with HindIII and PstI, ligated with T4 ligase to HindIIII, PstI digested pMYC031 and transformed into E.coli strain JM103. The DNA from one transformant, M13MYC0102, (Figure 9) was digested with restriction endonucleases in order to derive the restriction map shown in Figure 9.

Based on the DNA sequence of the 74.5 kDa antigen gene a 36 base long synthetic oligonucleotide, COD 693 (see below) was designed to alter the 
204 TRP ASP ASN GLU ILE VAL ASN TRP LEU VAL LYS LYS ILE LYS
TGG GAT AAT GAA ATT GTA AAT TGA CTT GTT AAA AAA ATC AAA
CC CTA TTA CTT TAA CAT TTA ACC GAA CAA TTT TTT T
 TGA through site directed mutagenesis according to the methods of Taylor et al. (1985) Nucl Acid Res 13:8749-8764 and Taylor et al. (1985) Nucl Acid Res 13:8764-8785.

Single Strand DNA of M13MYC0102, prepared according to Nakamaye, K. and P. Eckstein Nucl Acid Res., Vol. 14, pgs 9679-9698 (1986), was used as the template in the steps shown in Figure 10, except that the oligocucleotide, COD693, contains only one mutation.

Four plaques were picked, their DNA prepared, and DNA sequence analysis confirmed that each had the desired TGA to TGG change. The TGG codon, like the TGA codon, encodes for tryptophan; however, unlike TGA, TGG is not recognized as a stop codon by E. coli. The RfDNA from all four phages was pooled and named M13MYC079, its restriction map proved to be identical to M13MYC0102.

In order to replace the normal 74.5 kDa antigen gene with its TGG codon counterpart, pMYC056 DNA was digested with StyI, treated with Calf Intestine Phospatase, ligated to StyI digested M13MYC079 with T4 ligase and transformed into E.coli strain CY15000. One transformant was named pMYC087; its restriction map proved to be identical to pMYC056. As 74.5 kDa antigen, pMYC087 allows peptide chain elongation at position 211 and produces an approximately 74 kDa protein.

In order to replace the ampicilllin resistance of pMYC087 with tetracycline resistance, pMYC087 DNA was digested with AflIII and EcoRI, ligated to the 2.5kb AflIII EcoRI fragment of the tetracycline resistance cloning vector pBR322 using T4 ligase, and transformed into E.coli strain MH1.

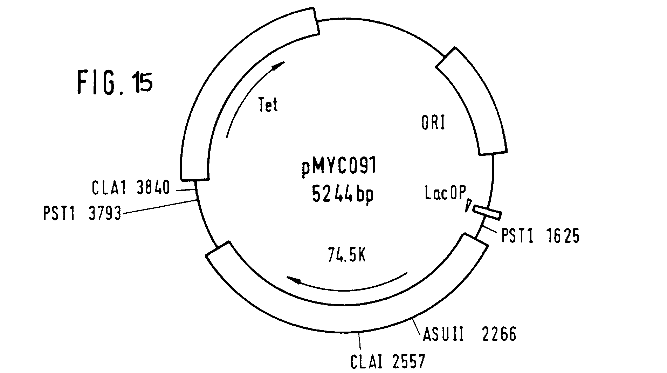

One transformant was named pMYC091 (Figure 14), its DNA prepared and digested with restriction enzymes to generate the map shown below. As expected, pMYC091produces an approximately 74 kDa protein. A map of the pMYC091 functional regions is presented in Figure 15.

It is to be noted that both pMYC087 and pMYC091 encode for the 74.5 kDa antigen in the absence of a TGA suppressor.

## Claims (Claims for the following Contracting State(s): AT, BE, CH, DE, FR, GB, GR, IT, LU, NL, SE)

1. An expression vehicle including a DNA sequence as shown in Figure 2, or a portion thereof, which encodes a protein which is capable of eliciting an antibody which recognises an epitope of the Mycoplasma hyopneumoniae 74.5 kDa antigen.

2. A vehicle according to claim 1, wherein the protein includes at least a portion of the sequence shown in Figure 2, except that Val¹⁷ is changed to Cys¹⁷ and Va1⁷ to Arg⁷.

3. A vehicle according to claim 1, which includes at least a portion of the DNA sequence shown in Figure 2, except that the codon TGA for amino-acid residue Trp¹¹ is changed to a TGG codon.

4. A vehicle according to any preceding claim, which additionally includes a DNA sequence which encodes the trp T 176 gene.

5. A host organism transformed with an expression vehicle according to any of claims 1 to 4.

## Claims (Claims for the following Contracting State(s): ES)

1. A process for preparing a transformed host organism, which comprises transforming the host with an expression vehicle including a DNA sequence as shown in Figure 2, or a portion thereof, which encodes a protein which is capable of eliciting an antibody which recognises an epitope of the Mycoplasma hyopneumoniae 74.5 kDa antigen.

2. A process according to claim 1, wherein the protein includes at least a portion of the sequence shown in Figure 2, except that Val¹⁷ is charged to Cys¹⁷ and Val⁷ to Arg⁷.

3. A process according to claim 1, wherein the DNA sequence includes at least a portion of the DNA sequence shown in Figure 2, except that the codon TGA for amino-acid residue Trp¹¹ is changed to a TGG codon.

4. A process according to any preceding claim, wherein the DNA sequence additionally includes a DNA sequence which encodes the trp T 176 gene.

5. A process for preparing a protein as defined in claim 1 or claim 2, which comprises expression from a transformed host organism produced by a process according to any of claims 1 to 4.

## Patentansprüche (Patentansprüche für folgende(n) Vertragsstaat(en): AT, BE, CH, DE, FR, GB, GR, IT, LU, NL, SE)

1. Expressionsvehikel mit einer in Figur 2 dargestellten DNA-Sequenz oder einem Teil derselben mit Codierung für ein zur Produktion eines Antikörpers, der ein Epitop des 74,5 kDa großen Mycoplasma hyopneumoniae Antigens erkennt, fähiges Protein.

2. Vehikel nach Anspruch 1, wobei das Protein mindestens einen Teil der in Figur 2 dargestellten Sequenz, wobei jedoch Val¹⁷ in Cys¹⁷ und Va1⁷ in Arg⁷ geändert sind, umfaßt.

3. Vehikel nach Anspruch 1, welches mindestens einen Teil der in Figur 2 dargestellten DNA-Sequenz, wobei jedoch das Kodon TGA für den Aminosäurerest Trp¹¹ in ein Kodon TGG geändert ist, umfaßt.

4. Vehikel nach einem der vorhergehenden Ansprüche, das zusätzlich eine DNA-Sequenz mit Codierung für das trp T 176-Gen umfaßt.

5. Mit einem Expressionsvehikel nach einem der Ansprüche 1 bis 4 transformierter Wirtorganismus.

## Patentansprüche (Patentansprüche für folgende(n) Vertragsstaat(en): ES)

1. Verfahren zur Herstellung eines transformierten Wirtorganismus durch Transformieren des Wirts mit einem Expressionsvehikel mit einer in Figur 2 dargestellten DNA-Sequenz oder einem Teil derselben mit Codierung für ein zur Produktion eines Antikörpers, der ein Epitop des 74,5 kDa großen Mycoplasma hyopneumoniae Antigens erkennt, fähiges Protein.

2. Verfahren nach Anspruch 1, wobei das Protein mindestens einen Teil der in Figur 2 dargestellten Sequenz, wobei jedoch Val¹⁷ in Cys¹⁷ und Va1⁷ in Arg⁷ geändert sind, umfaßt.

3. Verfahren nach Anspruch 1, wobei die DNA-Sequenz mindestens einen Teil der in Figur 2 dargestellten DNA-Sequenz, wobei jedoch das Kodon TGA für den Aminosäurerest Trp¹¹ in ein Kodon TGG geändert ist, umfaßt.

4. Verfahren nach einem der vorhergehenden Ansprüche, wobei die DNA-Sequenz zusätzlich eine DNA-Sequenz mit Codierung für das trp T 176-Gen umfaßt.

5. Verfahren zur Herstellung eines Proteins nach Anspruch 1 oder 2 durch Expression aus einem nach einem Verfahren gemäß einem der Ansprüche 1 bis 4 hergestellten transformierten Wirtorganismus.

## Revendications (Revendications pour l'(les) Etat(s) contractant(s) suivant(s): AT, BE, CH, DE, FR, GB, GR, IT, LU, NL, SE)

1. Véhicule d'expression comprenant une séquence d'ADN telle qu'elle est représentée sur la figure 2, ou une portion de cette séquence, qui code pour une protéine qui est capable d'engendrer un anticorps qui reconnaît un épitope de l'antigène de 74,5 kDa de Mycoplasma hyopneumoniae.

2. Véhicule suivant la revendication 1, dans lequel la protéine comprend au moins une partie de la séquence représentée sur la figure 2, sauf que Val¹⁷ est remplacé par Cys¹⁷ et Val⁷ est remplacé par Arg⁷.

3. Véhicule suivant la revendication 1, qui comprend au moins une partie de la séquence d'ADN représentée sur la figure 2, sauf que le codon TGA pour le résidu d'aminoacide Trp¹¹ est remplacé par le codon TGG.

4. Véhicule suivant l'une quelconque des revendications précédentes, qui comprend en outre une séquence d'ADN qui code pour le gène trp T 176.

5. Organisme hôte transformé avec un véhicule d'expression suivant l'une quelconque des revendications 1 à 4.

## Revendications (Revendications pour l'(les) Etat(s) contractant(s) suivant(s): ES)

1. Procédé de préparation d'un organisme hôte transformé, qui comprend la transformation de l'hôte avec un véhicule d'expression comprenant une séquence d'ADN telle que représentée sur la figure 2, ou une partie de cette séquence, qui code pour une protéine qui est capable d'engendrer un anticorps qui reconnaît un épitope de l'antigène de 74,5 kDa de Mycoplasma hyopneumoniae.

2. Procédé suivant la revendication 1, dans lequel la protéine comprend au moins une partie de la séquence représentée sur la figure 2, sauf que Val¹⁷ est remplacé par Cys¹⁷ et Val⁷ est remplacé par Arg⁷.

3. Procédé suivant la revendication 1, dans lequel la séquence d'ADN comprend au moins une partie de la séquence d'ADN représentée sur la figure 2, sauf que le codon TGA pour le résidu d'aminoacide Trp¹¹ est remplacé par le codon TGG.

4. Procédé suivant l'une quelconque des revendications précédentes, dans lequel la séquence d'ADN comprend en outre une séquence d'ADN qui code pour le gène trp T 176.

5. Procédé de préparation d'une protéine répondant à la définition suivant la revendication 1 ou la revendication 2, qui comprend une expression par un organisme hôte transformé produit par un procédé suivant l'une quelconque des revendications 1 à 4.
